# EUROPEAN PATENT APPLICATION

(11) **EP 2 063 305 A2**
(43) Date of publication of application: **27.05.2009**
(21) Application number: 08019801.3
(22) Date of filing: 12.11.2008
(51) Int. Cl.: G02B 21/00, G02B 21/18, G02B 21/22

(54) **A surgical microscope apparatus**

(30) Priority: 13.11.2007 JP 2007294488
(71) Applicant: Kabushiki Kaisha Topcon, Tokyo (JP)
(72) Inventor: Machida, Kazutoshi, Tokyo (JP); Akiyama, Hiroshi, Tokyo (JP)
(74) Representative: Gagel, Roland

(57) **Abstract**

A surgical microscope apparatus 1 comprises an illumination optical system 20, an observation optical system 30, and a controller 60. The illumination optical system 20 has an illumination light source 21 configured to emit an illumination light, and an emission diaphragm 23 and illumination diaphragm 24 both configured to change the angle of the illumination light (illumination angle) with respect to the optical axis of the observation optical system 30. When the illumination angle is changed, the controller 60 determines the intensity of the illumination light based on the illumination angle after the change and illumination-intensity information 62a. Further, the controller 60 controls the illumination light source 21 so as to output an illumination light with the determined intensity. According to the surgical microscope apparatus 1, when the angle of an illumination light that illuminates an eye is changed, an observation image with favorable brightness can be easily obtained.

## Description

### BACKGROUND OF THE INVENTION

### 1. Field of the Invention

The present invention relates to a surgical microscope apparatus used in an ophthalmological surgery.

### 2. Description of the Related Art

In a surgery in the ophthalmological field, a microscope dedicated for observing a magnified image of an eye is used. Here, red reflex (a diaphanoscopy image) is known as a technique for enhancing the visibility of an observation image. A red reflex image is a reddish image obtained from a diffuse reflection light as a result of projection of an illumination light onto the fundus oculi of an eye (e.g., refer to Japanese unexamined patent application publication No. 08-257037).

A surgical microscope apparatus has an illumination optical system for illuminating an eye, and an observation optical system for observing an illuminated eye. The observation optical system is placed so that an optical axis thereof (observation optical axis) substantially coincides with the optical axis of the eye. The illumination optical system illuminates the eye from a direction tilted from the observation optical axis. An angle formed by the illumination light entering the eye and the observation optical axis will be referred to as an illumination angle hereinafter.

Here, it is known that an observation image with good contrast is obtained by illuminating an eye only with components of small illumination angle. Therefore, a surgical microscope apparatus is provided with a diaphragm for blocking part of an illumination light (components of large illumination angle or components of small illumination angle) (e.g., refer to Japanese unexamined patent application publication No. 2004-139002).

However, according to a conventional surgical microscope apparatus, part of a light emitted by a light source is blocked to enhance the contrast of a red reflex image, with the result that the intensity (the light amount) of an illumination light projected onto an eye decreases. Consequently, the brightness of an observation image lowers, and the clarity thereof may be impaired.

It is possible to regulate the intensity of a light emitted by a light source in order to brighten an observation image. However, the conventional surgical microscope apparatus has a problem of a complicated operation because the intensity is manually regulated. Moreover, it is not easy, particularly for a non-expert, to adjust to proper intensity in accordance with an illumination angle, and an operation therefor is also complicated.

### SUMMARY OF THE INVENTION

The present invention was devised to solve the abovementioned problems, and an object of the present invention is to provide a surgical microscope apparatus that enables easy acquisition of an observation image with preferable brightness when the angle of an illumination light that illuminates an eye is changed.

In order to achieve the above object, a first aspect of the present invention provides a surgical microscope apparatus comprising: an illumination optical system including a light source emitting an illumination light, and configured to project an illumination light emitted from the light source onto an eye; an observation optical system including an ocular lens, and configured to guide the reflected light of the illumination light by the eye to the ocular lens, wherein: the illumination optical system includes a changing part configured to change an angle of the illumination light with respect to an optical axis of the observation optical system; and a controller is comprised, configured to change the intensity of an illumination light emitted from the light source when the angle of the illumination light is changed by the changing part, based on an angle formed after the change.

Further, a second aspect of the present invention provides the surgical microscope apparatus of the first aspect, wherein: the changing part includes a blocking part configured to change the angle of the illumination light by blocking part of the illumination light emitted from the light source; and the controller changes the intensity of the illumination light emitted from the light source when the angle of the illumination light is changed by the blocking part, based on an aspect of blocking the illumination light after the change.

Further, a third aspect of the present invention provides the surgical microscope apparatus of the second aspect, wherein: the controller includes a storage configured to previously store association information that associates the aspect of blocking the illumination light by the blocking part with the intensity of the illumination light, acquires intensity associated with the aspect of blocking the illumination light after the change based on the association information, and controls the light source so as to emit an illumination light with the acquired intensity.

Further, a fourth aspect of the present invention provides the surgical microscope apparatus of the second aspect, wherein: the illumination optical system includes a reflecting member that is placed near an optical axis of the observation optical system and that is configured to reflect the illumination light passed through the blocking part toward the eye; the blocking part includes a first blocking member on which a hole with a shape corresponding to the shape of a reflecting face of the reflecting member is formed, a second blocking member capable of blocking part of the hole, and a driver capable of continuously moving the second blocking member with respect to the hole; and the controller changes the intensity of the illumination light emitted from the light source when the second blocking member is moved by the driver, based on the position of the second blocking member after the movement.

Further, a fifth aspect of the present invention provides the surgical microscope apparatus of the fourth aspect, wherein: the controller includes a detector configured to detect the position of the second blocking member, and changes the intensity of the illumination light based on the detected position.

Further, a sixth aspect of the present invention provides the surgical microscope apparatus of the fourth aspect, wherein: the controller controls the driver so as to move the second blocking member and changes the intensity of the illumination light based on the content of the control.

Further, a seventh aspect of the present invention provides the surgical microscope apparatus of the fourth aspect, wherein: the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

Further, an eighth aspect of the present invention provides the surgical microscope apparatus of the fifth aspect, wherein: the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

Further, a ninth aspect of the present invention provides the surgical microscope apparatus of the sixth aspect, wherein: the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

Further, a tenth aspect of the present invention provides a surgical microscope apparatus comprising: an illumination optical system including a light source emitting an illumination light, and configured to project the illumination light emitted from the light source onto an eye; an observation optical system including an ocular lens, and configured to guide the reflected light of the illumination by the eye to the ocular lens, wherein: the illumination optical system includes a changing part configured to change an angle of the illumination light with respect to an optical axis of the observation optical system; the observation optical system includes a light receiver configured to receive part of the reflected light of the illumination light by the eye; and a controller is comprised, configured to change the intensity of the illumination light emitted from the light source when the angle of the illumination is changed by the changing part, based on a result of reception by the light receiver after the change.

Further, an eleventh aspect of the present invention provides the surgical microscope apparatus of the tenth aspect, wherein: the controller changes the intensity of the illumination light so that the intensity of the reflected light received by the light receiver is included within a specified range.

The surgical microscope apparatus according to the present invention is configured to automatically change the intensity of an illumination light emitted from the light source when the illumination angle is changed by the changing part, based on the angle after the change. Therefore, according to this surgical microscope apparatus, an observation image with favorable brightness can be easily obtained when the angle of an illumination light that illuminates an eye is changed.

Further, the surgical microscope apparatus according to the present invention is configured to automatically change the intensity of an illumination light emitted from the light source when the illumination angle is changed by the changing part, based on the result of reception of the reflected light of the illumination light by the eye obtained by the light receiver after the change. Therefore, according to this surgical microscope apparatus, it is possible to easily obtain an observation image with favorable brightness when the angle of an illumination light that illuminates an eye is changed.

### BRIEF DESCRIPTION OF THE DRAWINGS

Fig. 1 is a schematic view illustrating an example of the appearance of an embodiment of a surgical microscope apparatus according to the present invention.
Fig. 2 is schematic view illustrating an example of the appearance of the embodiment of the surgical microscope apparatus according to the present invention.
Fig. 3 is schematic view illustrating an example of the appearance of the embodiment of the surgical microscope apparatus according to the present invention.
Fig. 4 is a schematic view illustrating an example of the configuration of an optical system of the embodiment of the surgical microscope apparatus according to the present invention.
Fig. 5 is a schematic view illustrating an example of the configuration of the optical system of the embodiment of the surgical microscope apparatus according to the present invention.
Fig. 6 is a schematic view illustrating an example of the configuration of the optical system of the embodiment of the surgical microscope apparatus according to the present invention.
Fig. 7 is a schematic view illustrating an example of the configuration of the optical system of the embodiment of the surgical microscope apparatus according to the present invention.
Fig. 8 is a schematic view illustrating an example of the configuration of the optical system of the embodiment of the surgical microscope apparatus according to the present invention.
Fig. 9 is a schematic view illustrating an example of the configuration of the optical system of the embodiment of the surgical microscope apparatus according to the present invention.
Fig. 10 is a schematic block diagram illustrating an example of the configuration of a control system of the embodiment of the surgical microscope apparatus according to the present invention.
Fig. 11 is a flowchart illustrating an example of an operation aspect of the embodiment of the surgical microscope apparatus according to the present invention.

### DETAILED DESCRIPTION OF THE PREFERRED EMBODIMENTS

An embodiment of a surgical microscope apparatus according to the present invention will be described in detail with reference to the drawings.

### [APPEARANCE]

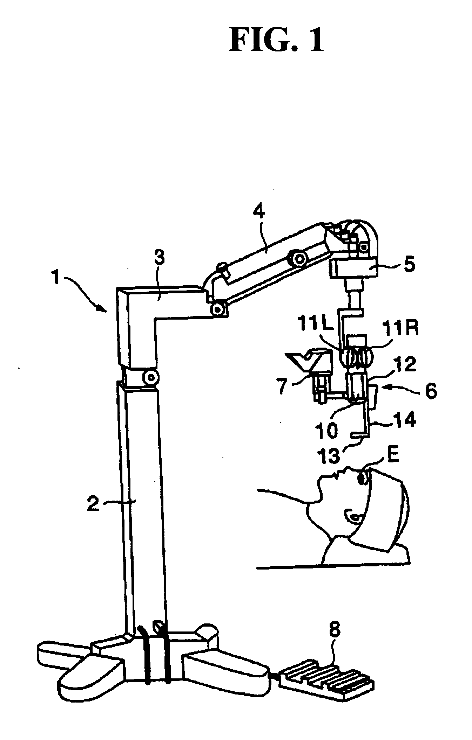

Firstly, with reference to Fig. 1 to Fig. 3, the appearance of the surgical microscope apparatus according to this embodiment will be described. A surgical microscope apparatus 1 is used to observe a surgical field in a surgery of an eye E.

One end of a first arm 3 is connected to the upper end of a supporting post 2 of the surgical microscope apparatus 1. One end of a second arm 4 is connected to the other end of the first arm 3. A drive unit 5 is connected to the other end of the second arm 4. An operator's microscope 6 is suspended from the drive unit 5. An assistant's microscope 7 is attached to the operator's microscope 6.

The surgical microscope apparatus 1 is provided with a foot switch 8. The operator makes the surgical microscope apparatus 1 operate a desired operation by operating the foot switch 8 with his/her foot. The drive unit 5 3-dimensionally moves the operator's microscope 6 and the assistant's microscope 7 in response to the operation using the foot switch 8, etc.

Various kinds of optical systems and drive systems are housed in a lens tube part 10 of the operator's microscope 6. An inverter part 12 is disposed at the upper part of the lens tube part 10. The inverter part 12 is an optical unit that converts a reverted image into an erected image. Right and left eyepieces 11R and 11L are disposed at the upper part of the inverter part 12. The eyepieces 11L and 11R are provided with ocular lenses, respectively (described later). Moreover, at the lower end of the lens tube part 10, an objective lens 15 is disposed.

The upper end of a holding arm 14 is connected to the operator's microscope 6. A head lens 13 is held at the lower end of the holding arm 14. The head lens 13 focuses an illumination light and illuminates the inside of the eye E. As the head lens 13, a plurality of lenses having different refractive powers (40D, 80D, 120D, etc.) are prepared, and selectively mounted on the holding arm 14.

The upper end of the holding arm 14 is pivotally disposed so as to be rotatable in the vertical direction. Thus, the head lens 13 can be inserted into and removed from a position between the eye E and the objective lens 15. A position where the head lens 13 is inserted (a using position) is a position on the optical axis of the objective lens 15, between the anterior focal position of the objective lens 15 and the eye E.

The head lens 13 is held by a holding plate 141 a formed so as to encompass it. The holding plate 141a is connected to an arm part 141 via an axis 141b, and is rotatable around the axis 141b. A slanting part 141 c is formed on the holding plate 141 a.

A coil spring 154 is wound around the upper end of the arm part 141. The upper end of the arm part 141 is pivotally disposed to one end of a housing part 174 by an axis 174a. The arm part 141 is provided with an operation knob extending horizontally when seen from the operator's side (not shown). By grasping this operation knob and swiveling the holding arm 14 around the axis 174a, the operator can switch the position of the head lens 13 between the using position described above and a housing position described later.

A main body 6a of the operator's microscope 6 has a driver 175.

To the driver 175, an up-and-down arm 171 is connected via a supporting member 176. At the upper end of the up-and-down arm 171, a fringe part 171 a is formed, whereby the up-and-down arm 171 is prevented from falling off the supporting member 176. The driver 175 moves the up-and-down arm 171 in the vertical direction, together with the supporting member 176. At this moment, together with the up-and-down arm 171, the head lens 13 is also moved integrally.

A connecting part 171b is connected to the lower end of the up-and-down arm 171. An elevation restraining member 172 is connected to the connecting part 171b. The elevation restraining member 172 contacts an elevation restraining member 177 on the side of the main body 6a when the up-and-down arm 171 is elevated to a specified position. Thus, the elevation restraining members 172 and 177 act so as to prevent the up-and-down arm 171 from moving more upward than the specified position.

A coupling knob 173 is disposed to the connecting part 171b.

When the coupling knob 173 is rotated in a specified direction, the tip of a rotation screw (not shown) is fitted into a coupling hole 177a. Consequently, the head lens 13, the holding arm 14, the housing part 174, etc., are coupled to the main body 6a. In this coupling state, movement of the head lens 13, etc. is inhibited.

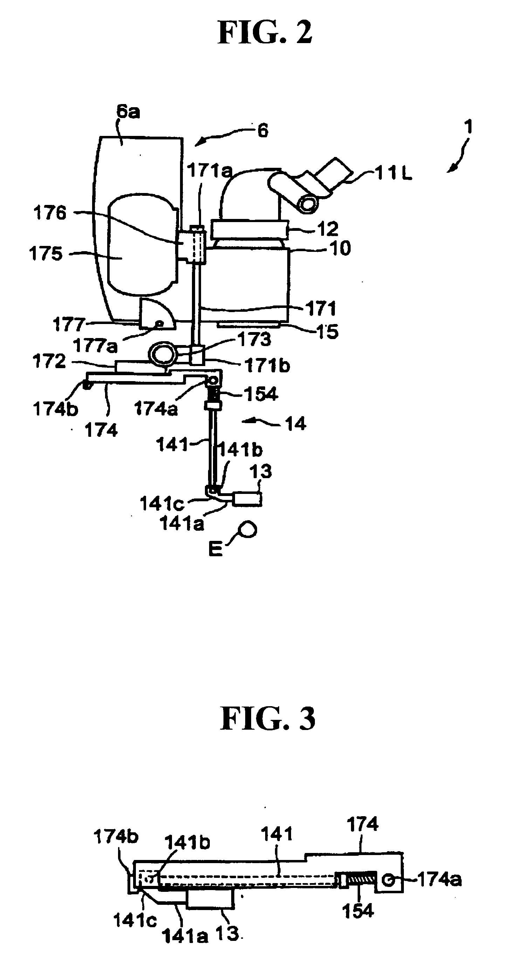

The housing part 174 is connected to the elevation restraining member 172. The housing part 174 houses the holding arm 14 (and the head lens 13). Fig. 3 shows a state in which the holding arm 14 is housed. On the lower face of the housing part 174, a concave housing part is formed along the longitudinal direction of the housing part 174.

The holding arm 14 is swiveled around the axis 174a to be housed into the housing part.

In the state in which the holding arm 14 is housed, as shown in Fig. 3, the lens faces of the head lens 13 are directed in the vertical direction. This is because of the action of the slanting part 141 c of the holding plate 141 a and a contacting member 174b attached to the end of the housing part 174. That is, when the arm part 141 is swiveled upward around the axis 174a, the slanting part 141c comes in contact with the contacting member 174b, and the holding plate 141a is guided along the slanting part 141 c to rotate around the axis 141 b.

Consequently, the head lens 13 is placed in the housing position in a state as shown in Fig. 3.

On the other hand, Fig. 2 shows a state where the head lens 13 is placed in the using position between the eye E and the objective lens 15. To house the head lens 13 from this state, the operator grasps the abovementioned operation knob and swivels the holding arm 14 upward, thereby housing the head lens 13 and the holding arm 14 into the housing part 174. On the contrary, it is possible to bring the housed head lens 13 into the usage state by swiveling the holding arm 14 downward.

The housing part 174 is formed so as to be attachable to and detachable from the elevation restraining member 172. This is for removing the head lens 13 and the holding arm 14 from the operator's microscope 6 when sterilizing them. A part from the housing part 174 to the head lens 13 is integrally composed. In a state in which the head lens 13, etc., are removed, the surgical microscope apparatus 1 can be used as a surgical microscope apparatus without the head lens 13.

### [CONFIGURATION OF OPTICAL SYSTEM]

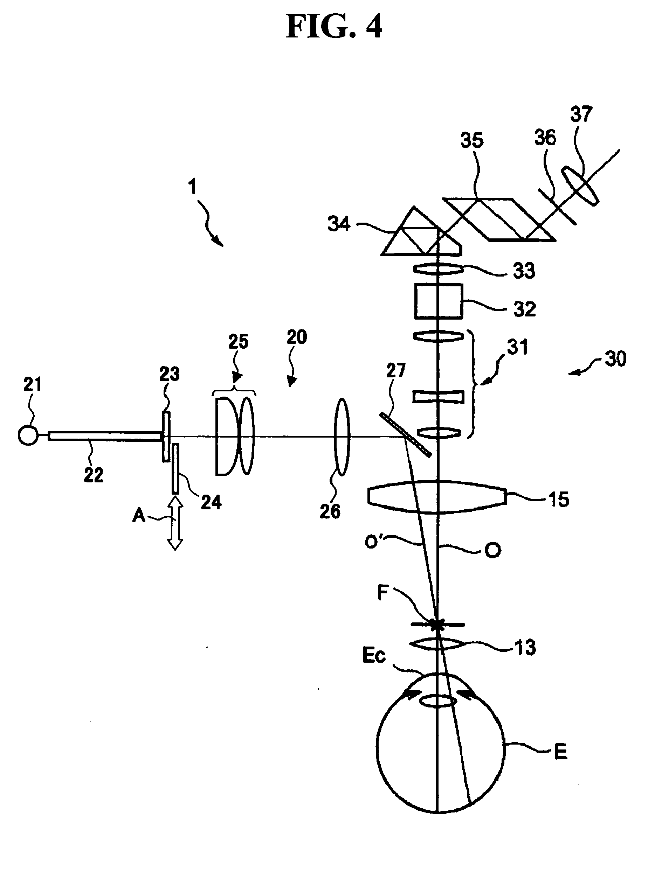

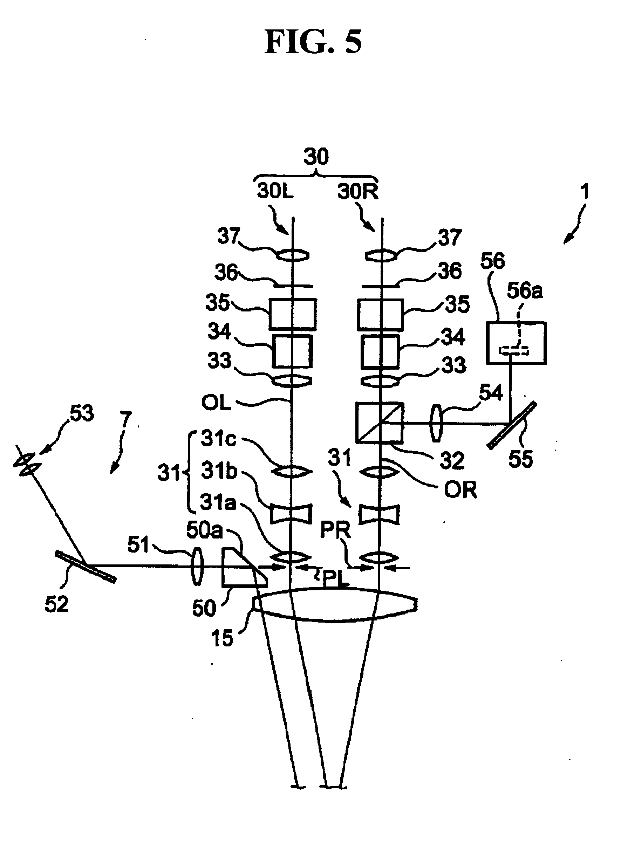

Next, with reference to Fig. 4 to Fig. 9, an optical system of the surgical microscope apparatus 1 will be described. Here, Fig. 4 is a view taken from the assistant's microscope 7, and Fig. 5 is a view taken from the operator. In this embodiment, the vertical direction, the horizontal direction, the anteroposterior direction, etc., are directions taken from the operator unless stated otherwise.

### [Observation Optical System]

As shown in Fig. 5, a pair of observation optical systems 30 are disposed. An observation optical system 30L on the left side will be referred to as a left observation optical system. An observation optical system 30R on the right side will be referred to as a right observation optical system. A symbol OL denotes the observation optical axis of the left observation optical system 30L. A symbol OR denotes the observation optical axis of the right observation optical system 30R.

The right and left observation optical systems 30R and 30L are formed so as to sandwich the optical axis O of the objective lens 15.

Each of the right and left observation optical systems 30R and 30L has a zoom lens system 31, a beam splitter 32 (only the right observation optical system 30R), an imaging lens 33, an image-erecting prism 34, an interpupillary adjustment prism 35, a field diaphragm 36, and an ocular lens 37. The zoom lens system 31 includes a plurality of zoom lenses 31 a, 31b and 31 c.

The beam splitter 32 of the right observation optical system 30R separates part of an observation light guided along the observation optical axis OR from the eye E, and guides it to a TV-camera imaging system. This TV-camera imaging system includes an imaging lens 54, a reflecting mirror 55, and a TV camera 56.

The TV camera 56 has an imaging device 56a. The imaging device 56a is composed of, for example, a CCD (Charge Coupled Device) image sensor, a CMOS (Complementary Metal Oxide Semiconductor) image sensor, etc. As the imaging device 56a, a device having a 2-dimensional light-receiving face, for example.

When the surgical microscope apparatus 1 is in use, the light-receiving face of the imaging device 56a is placed, for example, at a position optically conjugate to the surface of a cornea Ec, or a position optically conjugate to a position only a half of the corneal curvature radius distant from the corneal apex in the depth direction.

The assistant's microscope 7 is a microscope used by an assistant who assists the operator. The assistant's microscope 7 is provided with an optical system forming right and left observation light paths through the objective lens 15. This optical system includes a prism 50, an imaging lens 51, a reflecting mirror 52, and an ocular lens 53, as shown in Fig. 4.

The prism 50 is disposed near the circumferential end of the objective lens 15. The observation light from the eye E is propagated through the objective lens 15 to enter the prism 50 and is reflected by a reflecting face 50a. The observation light is then focused by the imaging lens 51, reflected by the reflecting mirror 52; and guided to the ocular lens 53. An entrance pupil of the optical system of the assistant's microscope 7 is the reflecting face 50a of the prism 50.

### [Illumination Optical System]

An illumination optical system 20 includes an illumination light source 21, a light guide 22, an emission diaphragm 23, an illumination diaphragm 24, a condenser lens 25, a collimator lens 26, and a mirror 27 as shown in Fig. 4.

The illumination light source 21 is an example of the "light source" of the present invention, and outputs an illumination light for illuminating the eye E. The illumination light source 21 is disposed, for example, outside the lens tube part 10. The illumination light source 21 includes, for example, an arbitrary luminescent device such as a halogen lamp, a xenon lamp, and an LED (Light Emitting Diode).

The illumination light source 21 may include a power supply unit for supplying electricity to this luminescent device.

The light guide 22 includes an optical fiber, etc. One end face of the light guide 22 is disposed near the illumination light source 21. A light outputted from the illumination light source 21 (or part of the light) enters the end face (entrance aperture) and is guided to the other end face (emission aperture).

An emission diaphragm 23 is disposed at a position facing the emission aperture of the light guide 22. The emission diaphragm 23 blocks a partial region of the emission aperture of the light guide 22.

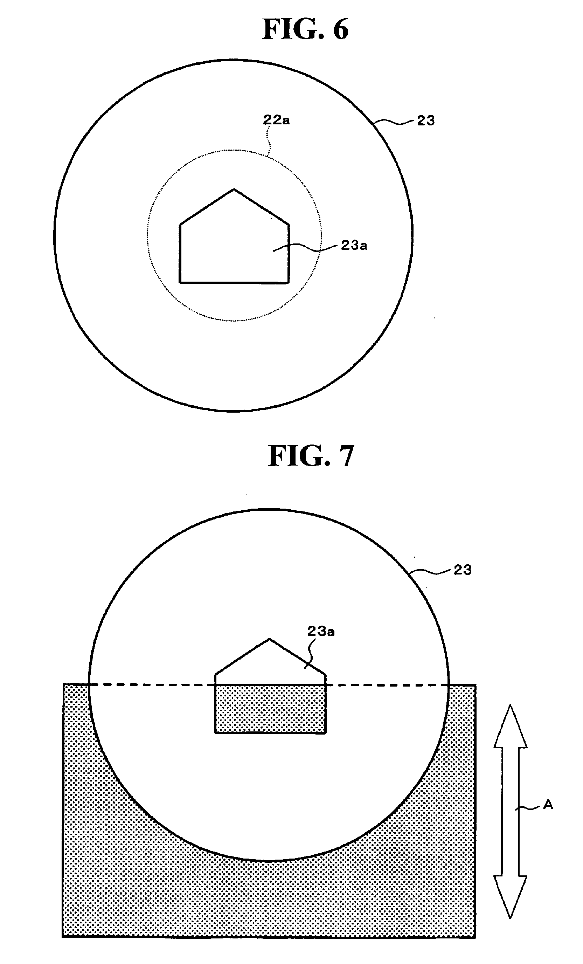

As shown in Fig. 6, a pentagonal hole 23a is formed on the emission diaphragm 23. The hole 23a is smaller than the emission aperture 22a of the light guide 22. The shape of the hole 23a is formed in accordance with the shape of the reflecting face of the mirror 27 (described later).

An illumination diaphragm 24 is disposed near the emission diaphragm 23. As shown in Fig. 7, the illumination diaphragm 24 blocks part of the hole 23a of the emission diaphragm 23. Furthermore, the illumination diaphragm 24 is movable relatively to the emission diaphragm 23. The direction of the movement is a direction shown by arrow A in Figs. 4 and 7. The illumination diaphragm 24 is continuously movable, particularly with respect to the emission diaphragm 23 (it may be movable in steps). With this illumination diaphragm 24, it is possible to change the aperture region of the hole 23a.

The emission diaphragm 23 is an example of the "first blocking member," and the illumination diaphragm 24 is an example of the "second blocking member." The position of the emission diaphragm 23 and the position of the illumination diaphragm 24 may be reserved.

The condenser lens 25 focuses the illumination light propagated through the emission diaphragm 23 and the illumination diaphragm 24.

The collimator lens 26 collimates the illumination light focused by the condenser lens 25. The collimated illumination light travels toward the mirror 27.

The mirror 27 is placed so that a reflecting face thereof slants toward the traveling direction of the illumination light. This reflecting face is a face of the mirror 27 on the side of the collimator lens 26.

This reflecting face has a shape corresponding to the hole 23a of the emission diaphragm 23 as mentioned above. The mirror 27 is an example of the "reflecting member" of the present invention. Instead of the mirror 27, a reflecting member such as a prism having a reflecting face for reflecting an illumination light can be used.

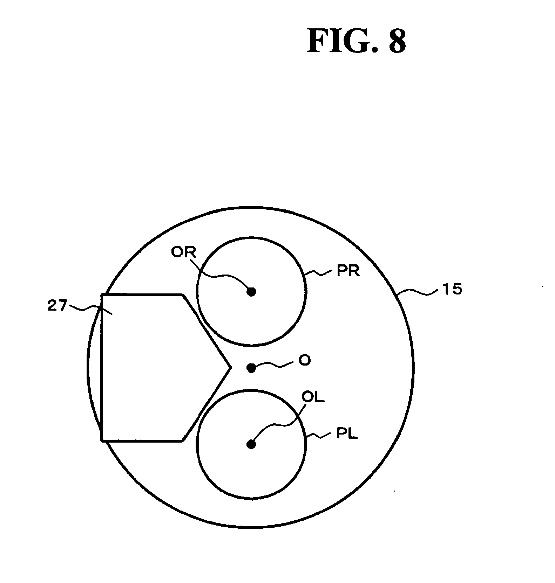

The positional relationship between the illumination optical system 20 and the observation optical system 30 is shown in Fig. 8.

This view illustrates an aspect of the objective lens 15, etc., when seen from above. The mirror 27 (the reflecting face thereof) is formed into a pentagonal shape like the hole 23a of the emission diaphragm 23. This pentagonal base is located above the apex thereof and closer to the optical axis O. Additionally, the oblique sides of the mirror 27 (two sides towards the base with the apex as an end) have a shape that prevents the mirror 27 from blocking a light entering the observation optical system 30. Here, a symbol PL denotes an entrance pupil of the left observation optical system 30L, and a symbol PR denotes an entrance pupil of the right observation optical system 30R (refer to Fig. 5).

The shape of the mirror 27 and the shape of the hole 23a are reversed because of design in which an illumination light is focused once between the condenser lens 25 and the collimator lens 26.

An exit pupil of the illumination optical system 20 is formed on the reflecting face of the mirror 27. When the illumination diaphragm 24 does not block the hole 23a at all, a pentagonal exit pupil is formed almost all over the entire reflecting face of the mirror 27. On the other hand, when the illumination diaphragm 24 blocks part of the hole 23a, an exit pupil corresponding to the region of the hole 23a that is not blocked is formed on the reflecting face.

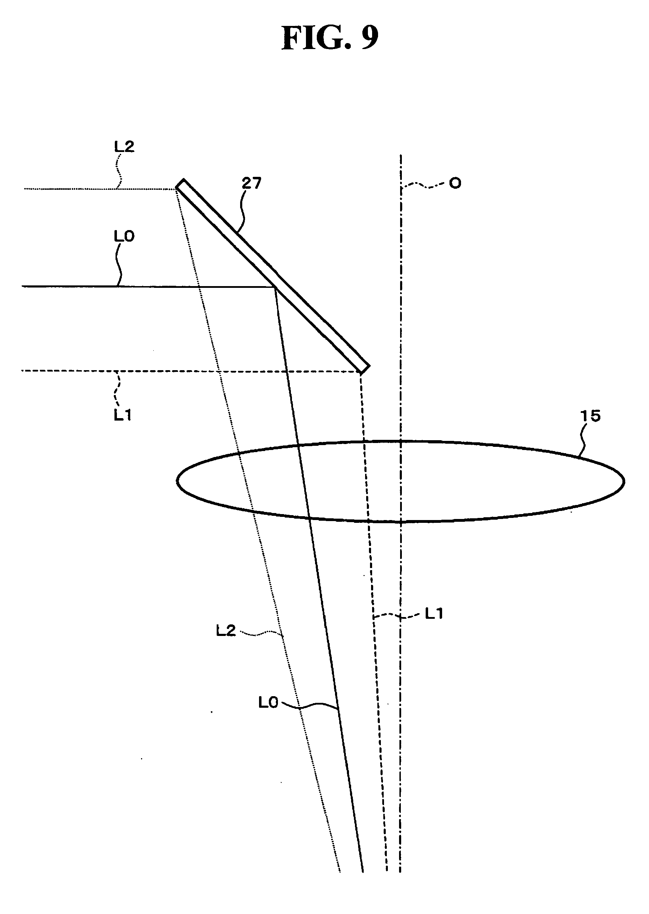

Here, an illumination angle will be described with reference to Fig. 9. The illumination angle is an angle formed by an illumination light projected onto the eye E and the optical axis O (parallel to the optical axes OL and OR of the observation optical system 30) of the objective lens 15.

The illumination angle can be changed by changing the position of the illumination diaphragm 24. When the illumination diaphragm 24 does not block the region of the hole 23a, the illumination light is applied onto and reflected by the (almost) entire mirror 27. That is, in this case, an illumination light composed of a beam having a region sandwiched by a symbol L1 and a symbol L2 as a cross-section shown in Fig. 9 is used.

Here, a beam of a region sandwiched by a symbol L0 and the symbol L1 represents, for example, components with the illumination angle of 2° (2° illumination components). Additionally, a beam of a region sandwiched by the symbol L0 and the symbol L2 represents, for example, components with the illumination angle of 4° (4° illumination components).

Additionally, when the illumination diaphragm 24 blocks a specified region on the base side of the hole 23a (e.g., a region lower than half of the height of the pentagon, which is the distance from the base to the apex), the illumination light is applied onto and reflected by only a specified region on the lower end of the mirror 27. That is, in this case, an illumination light composed of only 2° illumination components is used. This illumination aspect is applied to, for example, an observation using red reflex.

Since the illumination diaphragm 24 is continuously movable as mentioned above, it is possible to implement an illumination aspect other than the above as necessary.

### [CONFIGURATION OF CONTROL SYSTEM]

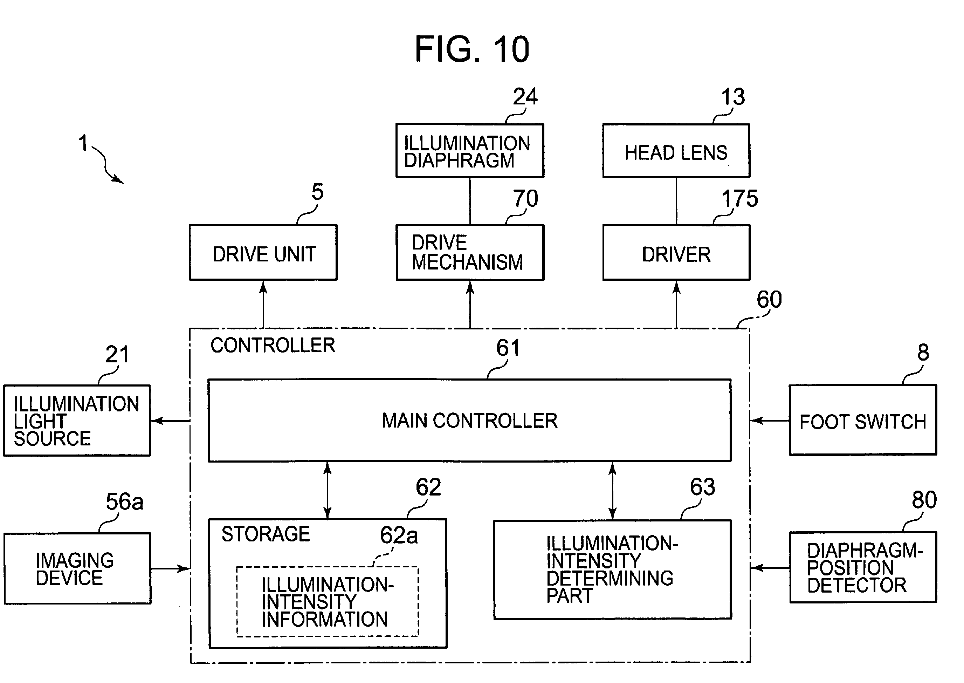

Next, the control system of the surgical microscope apparatus 1 will be described with reference to Fig. 10.

### [Controller]

The control system of the surgical microscope apparatus 1 is configured centering on a controller 60. The controller 60 controls each part of the surgical microscope apparatus 1. Additionally, the controller 60 executes various kinds of data processing. The controller 60 is an example of the "controller" of the present invention.

The controller 60 includes a microprocessor such as a CPU and a storage device such as a RAM, a ROM, and a hard disk drive. A program for controlling the surgical microscope apparatus 1 is previously stored in this storage device.

A main controller 61 controls the abovementioned drive unit 5, driver 175, and illumination light source 21, respectively. Moreover, when the foot switch 8 is operated, the main controller 61 controls the surgical microscope apparatus 1 so as to execute a movement corresponding to the content of the operation.

Further, the main controller 61 receives the result of detection by the imaging device 56a. The main controller 61 then controls a display device (not shown) to display a captured image of the eye E based on this detection result. Additionally, the main controller 61 controls a storage device such as a storage 62 to store this detection result (a captured image, etc.).

Furthermore, the main controller 61 controls a drive mechanism 70. The drive mechanism 70 is controlled by the main controller 61 to move the illumination diaphragm 24. The drive mechanism 70 includes an actuator such as a motor. The drive mechanism 70 is an example of the "driver" of the present invention.

The illumination diaphragm 24 may be configured to be moved manually. In this case, for example, an operation knob is disposed to (the lens tube part 10 of) the operator's microscope 6. When the operation knob is operated, the drive mechanism transmits a drive force thereof to the illumination diaphragm 24. This drive mechanism includes, for example, a gear. The operation knob and the drive mechanism are examples of the "driver" of the present invention.

The abovementioned driver configures examples of the "blocking part" and the "changing part" of the present invention together with the diaphragms 23 and 24.

Furthermore, the result of detection by a diaphragm-position detector 80 is inputted into the main controller 61. The diaphragm-position detector 80 detects the position of the illumination diaphragm 24. The diaphragm-position detector 80 includes an arbitrary position-detecting device such as a potentiometer.

The storage 62 stores various kinds of data. The storage 62 includes a storage device such as a hard disk drive. The storage 62 is an example of the "storage" of the present invention.

The storage 62 previously stores illumination-intensity information 62a. The illumination-intensity information 62a is information that associates an aspect of blocking an illumination light by the illumination diaphragm 24 (and the emission diaphragm 23) with the intensity of the illumination light. The illumination-intensity information 62a is an example of the "association information" of the present invention.

A specific example of the illumination-intensity information 62a will now be described. If the intensity of the illumination light is constant, the intensity of the illumination light projected onto the eye E changes according to the position of the illumination diaphragm 24, i.e., the position of the illumination diaphragm 24 relative to the emission diaphragm 23. In other words, when the region of the hole 23a blocked by the illumination diaphragm 24 is increased, the intensity of the illumination light projected onto the eye E decreases, and when the blocked region is decreased, the intensity of the illumination light projected onto the eye E increases. Therefore, on the abovementioned assumption, the brightness of an observation image of the eye E changes according to the position of the illumination diaphragm 24.

Thus, when the brightness of an observation image significantly changes with change of the illumination angle, there is a fear that observation cannot be performed favorably. In particular, when the illumination diaphragm 24 largely blocks the hole 23a (e.g., for obtaining a red reflex image), there is a fear that the observation image becomes darker and the eye E cannot be observed in detail.

The illumination-intensity information 62a is information that associates the intensity of an illumination light with each position of the illumination diaphragm 24. This intensity of an illumination light represents the intensity of the illumination light emitted from the illumination light source 21. For example, association using the illumination-intensity information 62a is association of the position of the illumination diaphragm 24 with the intensity of the illumination light so that the intensity of the illumination light projected onto the eye E becomes (nearly) constant, regardless of the position of the illumination diaphragm 24.

In other words, the position of the illumination diaphragm 24 corresponds to a region to be blocked of the hole 23a on one-to-one basis. This blocked region corresponds to a cross-section of the illumination light reaching the mirror 27 on one-on-one basis.

Therefore, the position of the illumination diaphragm 24 corresponds to the cross-sectional area of the illumination light reaching the mirror 27 on one-on-one basis. If the distribution of the light intensity on the cross-section of the illumination light is uniform, it is possible to create the illumination-intensity information 62a that the cross-sectional area of the illumination light reaching the mirror 27 is inversely proportional to the intensity of the illumination light emitted from the illumination light source 21, based on the correspondence relationship. The inverse proportional relationship may be corrected based on actually measured values of the abovementioned intensity distribution.

An illumination-intensity determining part 63 determines the intensity of the illumination light based on the result of detection by the diaphragm-position detector 80, i.e., the detection result of the position of the illumination diaphragm 24, and the illumination-intensity information 62a. This process is executed by obtaining the intensity of the illumination light corresponding to the detection result of the position of the illumination diaphragm 24, with reference to the illumination-intensity information 62a.

In a case where the illumination-intensity information 62a associates the position of the illumination diaphragm 24 with the intensity of the illumination light as a continuous relationship (graph, etc.), the illumination-intensity determining part 63 searches the intensity of the illumination light corresponding to the detection result of the position of the illumination diaphragm 24 from the illumination-intensity information 62a.

On the other hand, in a case where the illumination-intensity information 62a associates the position of the illumination diaphragm 24 with the intensity of the illumination light as a discrete relationship (table information, simple plot information, etc.), and the detection result of the position of the illumination diaphragm 24 is not directly associated by the abovementioned discrete relationship, the illumination-intensity determining part 63 interpolates and obtains the intensity of the illumination light corresponding to the detection result based on the abovementioned discrete relationship.

### [OPERATION ASPECT]

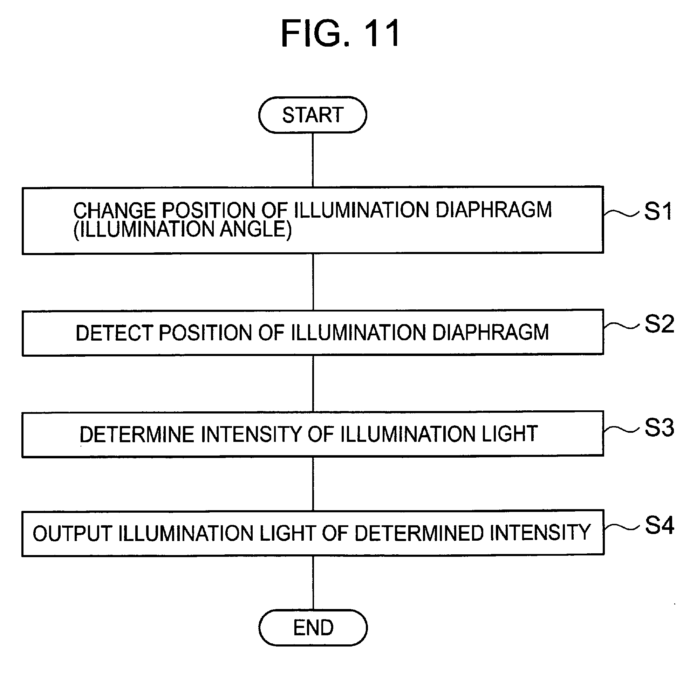

An operation aspect of the surgical microscope apparatus 1 will now be described. A flowchart shown in Fig. 11 represents an example of the operation aspect of the surgical microscope apparatus 1.

Firstly, an operator such as a surgical operator performs a specified operation to change the position of the illumination diaphragm 24 (S1). Consequently, the illumination angle with respect to the eye E is changed.

The diaphragm-position detector 80 detects the position of the illumination diaphragm 24 (S2). The diaphragm-position detector 80 may be configured to monitor the position of the illumination diaphragm 24 at all times, or may be configured to detect in response to change of the position of the illumination diaphragm 24. In the former case, it can be configured to detect the position of the illumination diaphragm 24 at a specified time interval, for example. In the latter case, it can be configured so that an operation signal is transmitted to the controller 60 from the foot switch 8 or knob operated by the operator, the controller 60 having received this operation signal sends a control signal to the diaphragm-position detector 80, and the diaphragm-position detector 80 having received this control signal detects the position of the illumination diaphragm 24, for example.

The diaphragm-position detector 80 sends the result of detection of the position of the illumination diaphragm 24 to the controller 60.

The illumination-intensity determining part 63 determines the intensity of the illumination light based on the detection result and the illumination-intensity information 62a (S3).

The main controller 61 controls the illumination light source 21 so as to output an illumination light with the intensity determined in Step 3 (S4). This control is executed by, for example, changing the value of current supplied to the abovementioned luminescent device (a halogen lamp, etc.) of the illumination light source 21 or the value of voltage to be applied to the luminescent device. With the above steps, the operation of automatically changing the intensity of the illumination light ends.

A specific example of the abovementioned operation aspect will now be described. The surgical operator first observes the eye E by using an entire illumination (an aspect of illumination using both the 2° illumination components and 4° illumination components).

Subsequently, the surgical operator operates the foot switch 8 and the knob to switch to the 2° illumination (S1) in order to observe by using red reflex.

The diaphragm-position detector 80 detects the position of the illumination diaphragm 24 after the switching (S2). This detection result is sent to the controller 60. The illumination-intensity determining part 63 acquires the intensity of the illumination light associated with the detection result (a position corresponding to the 2° illumination), with reference to the illumination-intensity information 62a (S3). The illumination light source 21 emits an illumination light with the intensity corresponding to the 2° illumination in response to the control of the main controller 61 (S4).

The surgical operator observes the eye E in this illumination state and adjusts the brightness of the observation image as needed.

This is because there are individual differences in condition of observation, depending on the condition of the eye E (opacity, pupil diameter, etc.) and the condition of the eyes of the surgical operator (eyesight, etc.). The brightness of the observation image is adjusted by, for example, operating the foot switch 8, etc., to increase/decrease the intensity of the illumination light emitted from the illumination light source 21. Additionally, similarly to Step 1, it is also possible to operate the foot switch, etc., to adjust the position of the illumination diaphragm 24. In this case, the surgical microscope apparatus 1 executes Step 2 through Step 4 again. Thus, the operator can adjust the condition of observation of the eye E as necessary.

### [ACTION/EFFECT]

The action and effect of the surgical microscope apparatus 1 according to this embodiment will now be described.

The surgical microscope apparatus 1 is provided with a function of changing an illumination angle. Furthermore, the surgical microscope apparatus 1 acts so as to change the intensity of an illumination light emitted from the illumination light source 21 when the illumination angle is changed, based on an angle after the change.

In particular, the surgical microscope apparatus 1 acts so as to change the illumination angle by blocking part of the illumination light emitted from the illumination light source 21, and then change the intensity of the illumination light emitted from the illumination light source 21 based on the aspect of blocking the illumination light (i.e., which part of the cross-sectional region of the illumination light is blocked).

Furthermore, the surgical microscope apparatus 1 acts so as to: previously store the illumination-intensity information 62a that associates the aspect of blocking the illumination light with the intensity of the illumination light; acquire the intensity associated with the aspect of blocking the illumination light based on the illumination-intensity information 62a; and control the illumination light source 21 so as to emit an illumination light with this acquired intensity.

Additionally, the aspect of blocking the illumination light (the illumination angle) is changed by the emission diaphragm 23 and the illumination diaphragm 24. The surgical microscope apparatus 1 detects the aspect of blocking the illumination light by detecting the position of the illumination diaphragm 24 by using the diaphragm-position detector 80, and obtains the intensity of the illumination light based on this detection result and the illumination-intensity information 62a.

According to the surgical microscope apparatus 1, when the aspect of blocking the illumination light (the illumination angle) is changed, it is possible to automatically determine the intensity of the illumination light corresponding to the blocking aspect after the change, and automatically output an illumination light with this intensity. Therefore, it is possible to easily obtain an observation image with proper brightness corresponding to the illumination angle.

Additionally, the surgical microscope apparatus 1 is configured to be capable of continuously changing the illumination angle (the blocked region of the illumination light) by the fixedly placed emission diaphragm 23 and the continuously movable illumination diaphragm. In the conventional surgical microscope apparatus, the illumination angle is changed in steps by using a turret plate having a plurality of holes.

However, with such a configuration, the operator cannot change the illumination angle freely. In particular, with the conventional configuration, it is impossible to finely adjust the illumination angle between steps. On the other hand, the configuration according to this embodiment enables continuous change of the illumination angle, and therefore, has a merit such that the operator can freely adjust the illumination angle.

### [MODIFICATION]

The configuration described in detail above is merely an example of the embodiment of the surgical microscope apparatus according to the present invention. Therefore, it is possible to apply an arbitrary modification as necessary within the scope of the present invention. Such a modification will be described below. The configuration of the embodiment above will be referred to as necessary in the following description.

### [First Modification]

Firstly, a surgical microscope apparatus that can determine the intensity of an illumination light without detecting the position of the illumination diaphragm 24 when the illumination diaphragm 24 is moved will be described. In the case of applying this modification, it is not necessary to dispose the diaphragm-position detector 80 shown in Fig. 10.

This modification is applicable when the controller 60 (the main controller 61) controls the drive mechanism 70 so as to move the illumination diaphragm 24. In this case, the controller 60 sends a control signal to the drive mechanism 70, thereby controlling the drive mechanism 70 so as to move the illumination diaphragm 24. This control signal may contain, for example, a pulse signal. The drive mechanism 70 may be provided with, for example, a stepping motor that rotates by an angle corresponding to the number of pulses of this pulse signal.

In this configuration, the controller 60 can specify the position of the illumination diaphragm 24. For example, the controller 60 can store the position of the illumination diaphragm 24 before this movement and, based on the position and the control signal, specify the position of the illumination diaphragm 24 after this movement.

The position of the illumination diaphragm 24 before the movement is specified, for example, in the following manner. When the surgical microscope apparatus 1 is powered on, the apparatus is initiated to move the illumination diaphragm 24 to a specified default position. When the illumination diaphragm 24 is moved after the initiation, displacement of the illumination diaphragm 24 from the default position, i.e., the position of the illumination diaphragm 24 after the movement, can be specified based on the default position and the control signal (the number of pulses). Storage of the position after this movement makes it possible to specify the position of the illumination diaphragm 24 after the movement similarly when the illumination diaphragm 24 is moved next time.

In a case where the diaphragm-position detector 80 is disposed, by detecting and storing the position of the illumination diaphragm 24 at a specified timing (e.g., at the time of power on) and executing a process similar to the above based on the detected position, it is possible to specify the position of the illumination diaphragm 24 after the movement.

According to this modification, when the illumination angle is changed, it is possible to easily obtain an observation image with proper brightness as in the surgical microscope apparatus 1 according to the above embodiment.

### [Second Modification]

In this modification, a surgical microscope apparatus configured to automatically change the intensity of an illumination light based on the reflected light of the illumination light from the eye E instead of the position of the illumination diaphragm 24 will be described.

The surgical microscope apparatus according to this modification has the illumination optical system 20 and the observation optical system 30 similar to those of the above embodiment. In particular, the illumination optical system 20 is provided with the emission diaphragm 23 and the illumination diaphragm 24, whereby it is possible to change the angle of the illumination light with respect to the optical axes OL and OR (parallel to the optical axis O of the objective lens 15) of the observation optical system 30.

Further, the observation optical system 30 is provided with the imaging device 56a as a light receiver that receives part of the reflected light of the illumination light from the eye E. The result of reception by the imaging device 56a is transmitted to the controller 60 (refer to Fig. 10).

When the angle of the illumination light is changed, the controller 60 receives the result of reception by the imaging device 56a after the change. The imaging device 56a may be configured to monitor the reflected light of the illumination light at all times, or may be configured to receive the reflected light in response to change of the angle of the illumination light. In the former case, the imaging device 56a is configured to receive the reflected light at a specified time interval, for example. In the latter case, the imaging device 56a can be configured to receive a control signal sent from the controller 60 having detected change of the illumination angle and receive the reflected light, for example.

The illumination-intensity information 62a different from that of the above embodiment is previously stored in the storage 62. The illumination-intensity information 62a according to this modification is, for example, information that associates the intensity of the reflected light received by the imaging device 56a with the intensity of the illumination light. Considering a case where an individual difference is made in intensity of the reflected light depending on the condition of the eye E (opacity, reflectivity on the fundus oculi, etc.) through the intensity of the illumination light is constant, it is possible to correct the illumination-intensity information 62a by tentatively projecting an illumination light at the beginning of observation of the eye E and receiving the reflected light. The illumination-intensity information 62a contains, for example, the intensity of an illumination light such that the intensity of the reflected light received by the imaging device 56a is always included in a specified range (preferably, almost constant), regardless of the illumination angle.

The illumination-intensity determining part 63 determines the intensity of the illumination light based on the result of reception by the imaging device 56a (the reception intensity of the reflected light) and the illumination-intensity information 62a. The main controller 61 controls the illumination light source 21 so as to output an illumination light with the determined intensity.

According to this modification, it is possible to easily obtain an observation image with favorable brightness when the illumination angle is changed, as in the surgical microscope apparatus 1 according to the above embodiment.

### [Another Modification]

In the above embodiment, a surgical microscope apparatus provided with a head lens is described, but the configuration according to the present invention can also be applied to a surgical microscope apparatus without a head lens. Also in this case, it is possible to obtain similar action and effect to those in the above embodiment.

In the embodiment above, a surgical microscope apparatus in which the illumination angle can be continuously changed is specifically described in detail, but the configuration according to the present invention can also be applied to a surgical microscope apparatus configured to change the illumination angle in steps.

For example, in a surgical microscope apparatus that can be used by switching between total illumination and 2° illumination (illumination composed of 2° illumination components alone), it is possible to configure so as to determine the intensity of the illumination light and control the illumination light source when the illumination angle is switched, based on the illumination angle (position of the illumination diaphragm 24, etc.) after the change.

Further, it is possible to configure so as to receive the reflected light of the illumination light from the eye after the illumination angle is switched, and then determine the intensity of the illumination light and control the illumination light source, based on the result of the reception.

In the above embodiment, the illumination angle is changed by inserting and removing the illumination diaphragm 24 into and from a specified direction (i.e., from below) with respect to the fixedly placed emission diaphragm 23, but the configuration for changing the illumination angle is not limited thereto. For example, it is possible to configure the illumination diaphragm 24 to be inserted from both above and below, or it is possible to configure the illumination diaphragm 24 to be inserted from a horizontal direction. Furthermore, in the surgical microscope apparatus according to the present invention, the changing part that changes the illumination angle is arbitrarily configured.

## Claims

1. A surgical microscope apparatus, comprising:
an illumination optical system including a light source emitting an illumination light, and configured to project the illumination light emitted from the light source onto an eye;
an observation optical system including an ocular lens, and configured to guide the reflected light of the illumination light by the eye to the ocular lens, wherein:
the illumination optical system includes a changing part configured to change an angle of the illumination light with respect to an optical axis of the observation optical system; and
a controller is comprised, configured to change the intensity of an illumination light emitted from the light source when the angle of the illumination light is changed by the changing part, based on an angle formed after the change.

2. The surgical microscope apparatus according to Claim 1, wherein:
the changing part includes a blocking part configured to change the angle of the illumination light by blocking part of the illumination light emitted from the light source; and
the controller changes the intensity of the illumination light emitted from the light source when the angle of the illumination light is changed by the blocking part, based on an aspect of blocking the illumination light after the change.

3. The surgical microscope apparatus according to Claim 2, wherein:
the controller includes a storage configured to previously store association information that associates the aspect of blocking the illumination light by the blocking part with the intensity of the illumination light, acquires intensity associated with the aspect of blocking the illumination light after the change based on the association information, and controls the light source so as to emit an illumination light with the acquired intensity.

4. The surgical microscope apparatus according to Claim 2, wherein:
the illumination optical system includes a reflecting member that is placed near an optical axis of the observation optical system and that is configured to reflect the illumination light passed through the blocking part toward the eye;
the blocking part includes a first blocking member on which a hole with a shape corresponding to the shape of a reflecting face of the reflecting member is formed, a second blocking member capable of blocking part of the hole, and a driver capable of continuously moving the second blocking member with respect to the hole; and
the controller changes the intensity of the illumination light emitted from the light source when the second blocking member is moved by the driver, based on the position of the second blocking member after the movement.

5. The surgical microscope apparatus according to Claim 4, wherein:
the controller includes a detector configured to detect the position of the second blocking member, and changes the intensity of the illumination light based on the detected position.

6. The surgical microscope apparatus according to Claim 4, wherein:
the controller controls the driver so as to move the second blocking member and changes the intensity of the illumination light based on the content of the control.

7. The surgical microscope apparatus according to Claim 4, wherein:
the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

8. The surgical microscope apparatus according to Claim 5, wherein:
the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

9. The surgical microscope apparatus according to Claim 6, wherein:
the controller includes a storage configured to previously store association information that associates the position of the second blocking member with the intensity of the illumination light, acquires intensity associated with the position of the second blocking member after the movement based on the association information, and controls the light source so as to emit an illumination light with the acquired intensity.

10. A surgical microscope apparatus, comprising:
an illumination optical system including a light source emitting an illumination light, and configured to project the illumination light emitted from the light source onto an eye;
an observation optical system including an ocular lens, and configured to guide the reflected light of the illumination by the eye to the ocular lens, wherein:
the illumination optical system includes a changing part configured to change an angle of the illumination light with respect to an optical axis of the observation optical system;
the observation optical system includes a light receiver configured to receive part of the reflected light of the illumination light by the eye; and
a controller is comprised, configured to change the intensity of an illumination light emitted from the light source when the angle of the illumination is changed by the changing part, based on a result of reception by the light receiver after the change.

11. The surgical microscope apparatus according to Claim 10, wherein:
the controller changes the intensity of the illumination light so that the intensity of the reflected light received by the light receiver is included within a specified range.
